**Europäisches Patentamt**

**European Patent Office**

**Office européen des brevets**

(11) Publication number: **0 249 346**
**A2**

(12) **EUROPEAN PATENT APPLICATION**

(21) Application number: **87304338.4**

(22) Date of filing: **15.05.87**

(51) Int. Cl.³: **A 61 F 2/08**

(30) Priority: **15.05.86 GB 8611853**

(43) Date of publication of application:
**16.12.87 Bulletin 87/51**

(84) Designated Contracting States:
**AT BE CH DE ES FR GB GR IT LI NL SE**

(71) Applicant: **Beacon, Jonathan Paul**
**Sewell Manor Sewell**
**Dunstable Bedfordshire(GB)**

(84) Designated Contracting States:

(71) Applicant: **Wadey, Raymond Clarence Chance**
**18 Greatwoods**
**Edington Near Westbury Wiltshire(GB)**

(84) Designated Contracting States:

(72) Inventor: **Beacon, Jonathan Paul**
**Sewell Manor Sewell**
**Dunstable Bedfordshire(GB)**

(72) Inventor: **Wadey, Raymond Clarence Chance**
**18 Greatwoods**
**Edington Near Westbury Wiltshire(GB)**

(74) Representative: **Burrows, Anthony Gregory et al,**
**Haseltine Lake & Co. Hazlitt House 28 Southampton**
**Buildings Chancery Lane**
**London, WC2 1AT(GB)**

(54) A prosthetic ligamentary device.

(57) A prosthetic ligamentary device 1 consists of a hollow longitudinal first element 2 of a relatively inelastic material, for example a cross-woven cord of polyester, and containing a hollow second element 3 of relatively elastic material, for example a co-axial silicone rubber tube. When the first element 2 is subjected longitudinally to an anatomical tensile force it extends to a limited degree by transversely compressing the second element 3, whereupon the device 1 becomes inextensible by the force.

FIG.1.

<u>PROSTHETIC LIGAMENTARY DEVICE</u>                **0249346**

This invention relates to a prosthetic ligamentary device.

From an anatomical point of view, it is desirable for an artificial ligament, during extension, to have elasticity followed by rigidity.

A prosthetic ligamentary device has been suggested consisting of a relatively inelastic cord attached at one end to one end of a helical tension spring. In its application to a knee joint, the cord is anchored at its other end to the tibia and extends at its one end into a hole drilled through the condyle of the femur, and the other end of the spring is anchored to the femur outside the hole.

In use of the joint, the one end of the cord must move longitudinally in the hole, but this is disadvantageous, because the one end of the cord should preferably be immobile in the hole, particularly to allow growth of natural material into the cord if the latter is foraminous.

United States Patent 4255820 discloses an artificial ligament which is tubular in overall configuration with flared ends, which have a graduated pore density to permit controlled hard and soft tissue ingrowth, and a central portion which prevents tissue ingrowth. The central section of the ligament is formed so as to be elastic along its longitudinal axis to permit the needed elasticity in use, having an elongation factor of between 4 to 6% of its length. Inserted within the central section is a cylindrical core of Dacron, or cross-linked or vulcanized rubber, or high-density polyethylene. The core is effective to maintain the tubular shape of the ligament and to provide strain relief throughout the central section.

According to one aspect of the present invention, there is provided a prosthetic ligamentary device including a first means which is relatively inelastic longitudinally of the device, characterized in that the device also includes a second means which is relatively elastic transversely of the device, and that said first means, at said second means and in one longitudinal direction of said first means, diverges outwardly and then converges inwardly, so that the subjection of the first means to an anatomical tensile force increases the length of the first means against the action of the second means to a limited degree and thereupon said first means becomes virtually inextensible.

Owing to the invention, it is possible to arrange for the second means, and thus the extension of the device, to be sited at the free zone of the device, whereby both ends of the ligament can be immobile, and yet for the device, during extension, to have elasticity followed by rigidity.

Advantageously, the first means diverges and converges as aforesaid about the second means and the latter is compressed by the extending of the first means, so that the first means is extended until the second means can be compressed no further by the tensile force.

According to another aspect of the present invention, there is provided a cruciate ligament prosthesis, characterized in that the prosthesis comprises first and second prosthetic ligamentary devices a section of each of which, when subjected longitudinally to an anatomical tensile force, increases in length to a limited degree and thereupon becomes virtually inextensible, and tube means encircling these sections of the devices, fastened at the ends thereof to the devices at locations beyond the sections and of material which is extensible with said sections.

In order that the invention may be clearly understood and readily carried into effect, reference will now be made, by way of example, to the accompanying drawings, in which:-

Figure 1 shows a diagrammatic sectional view of a prosthetic ligamentary device,

Figure 2 is a diagrammatic sectional view of a modified version of the device,

Figure 3 shows a diagrammatic sectional view of another modified version of the device,

Figure 4 shows a diagrammatic side elevation of a cruciate ligament prosthesis including two devices each according to the version of Figure 1,

Figure 5 is a front elevation of a left knee joint and shows the prosthesis of Figure 4 as an anterior cruciate ligament prosthesis,

Figure 6 is a rear elevation of a left knee joint and shows the prosthesis of Figure 4 as a posterior cruciate ligament prosthesis, and

Figure 7 is a diagrammatic sectional elevation of an acute reconstruction prosthetic ligamentary device.

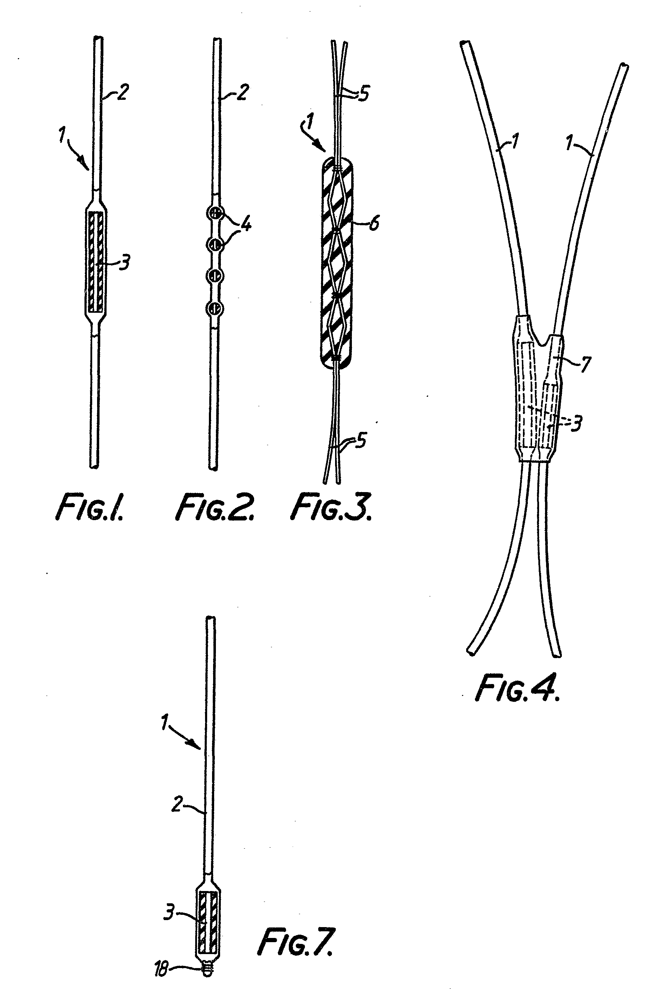

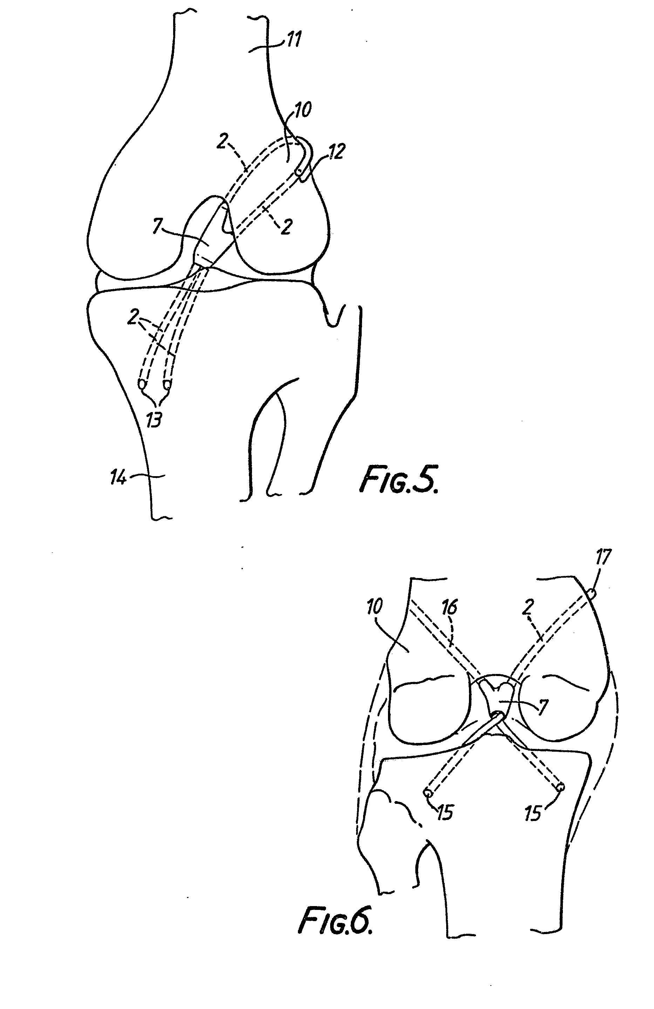

Referring to Figure 1, the prosthetic ligamentary device 1 consists of a hollow longitudinal element 2 of a relatively inelastic material and containing a hollow element 3 of relatively elastic material. The element 2 may be a cross-woven cord of polyester, whilst the element 3 may be a tube of silicone rubber co-axial with the element 2. When the element 2 is subjected longitudinally to an anatomical tensile force it extends to a limited degree by transversely compressing the tube 3, until the force is incapable of compressing the tube 3 further, whereupon the device 1 becomes inextensible by the force. By appropriate selection of the length and/or diameter of the tube 3, the degree of maximum extension of the device 1 can be preset.

In the version showing Figure 2, the hollow cord 2 contains spherical rubber beads 4 instead of the tube 3. Similarly, by appropriate selection of the number and diameter of the beads 4, the degree of maximum extension of the device can be preset.

Referring to Figure 3, there are two longitudinal elements 5 which zig-zag towards and away from each other in a silicone rubber body 6. By selecting the dimensions of the body 6 and/or the number and/or dimensions of the zig-zags therein, the degree of maximum extension of the device 1 can be preset. In manufacturing the version shown in Figure 3, the two cords 5 are passed in the zig-zags around pegs, their nodal zones are stitched together, and then the silicone rubber body 6 is cast around the zig-zag areas.

Referring to Figure 4, the prosthesis includes two devices 1 encircled by a bicornuate tube 7 which is fastened at its ends to the devices 1 at locations beyond the tubes 3 and which is of open-mesh material which is extensible with the devices.

Because the tubes 3 are of differing lengths in the prosthesis shown in Figure 4, differential maximum extension of the two devices 1 is achieved.

It will be noted with reference to Figures 5 and 6 that the extending of the cords 2 and the tube 7 occurs within the free zone of the prosthesis within the joint. This is important because extending elsewhere would cause the cords to slide within the drilled holes, thus discouraging ingrowth of natural body material.

Figure 5 shows an anterior application of the prosthesis of Figure 4. The Figure shows that the proximal part of one of the cords 2 has been passed posteriorly around the condyle 10 of the femur 11 to be attached to the adjacent end of the proximal part of the other cord 2, which has been passed through a hole 12 drilled through the condyle 10. The central unit 3, 7 has been twisted into position, with the distal parts of both cords 2 passing through holes 13 drilled in the tibia 14 and being fixed thereto.

Referring to Figure 6, showing a posterior application of the prosthesis of Figure 4, the distal parts of the cords 2 are passed through drilled holes 15 in the tibia 14 and are there fixed. The central unit 3, 7 of the prosthesis is again in a twisted condition.

This time, while the proximal part of one of the cords 2 is passed through a drilled hole 16 in the condyle 10 and there fixed, the proximal part of the other cord 2 is passed around the front of the condyle and is fixed at the location 17.

Where an acute reconstruction of a ligament is required, the device may be of the form shown in Figure 7, in which the cord 2 terminates just beyond one end of the tube 3 and is there provided with a fine cord whipping 18, with the end of the cord being heat-sealed to prevent flaying. The purpose of the fine cord whipping, which may be of polyester cotton, is to provide a sewing-in location for sutures to connect the device 1 to the damaged pole of the actual ligament.

An advantage of having the cord 2 and the tube 7 of woven material is that the interstices thereby provided promote ingrowth of natural body material.

The device 1 is deliberately implanted in a condition under tension for all positions of the knee and so is under continual tension _in situ_, owing to the relatively elastic element 3, and this predisposes towards the ingrowth of new living ligament material, with the intention of eventual production of a new biological ligament.

CLAIMS

1. A prosthetic ligamentary device including a first means (2,5) which is relatively inelastic longitudinally of the device (1), characterized in that the device (1) also includes a second means (3,4,6) which is relatively elastic transversely of the device (1), and that said first means (2,5), at said second means (3,4,6) and in one longitudinal direction of said first means (2,5), diverges outwardly and then converges inwardly, so that the subjection of the first means (2,5) to an anatomical tensile force increases the length of the first means (2,5) against the action of the second means (3,4,6) to a limited degree and thereupon said first means (2,5) becomes virtually inextensible.

2. A device according to claim 1, wherein said first means (2,5) diverges and converges as aforesaid about said second means (3,4,6) and said second means (3,4,6) is compressed by the increasing in length of said first means (2,5).

3. A device according to claim 2, wherein said first means (2)is a cross-woven hollow cord (2).

4. A device according to claim 2 or 3, wherein said second means (3,4,6) is a tube (3) substantially co-axial with said first means (2) and contained therein.

5. A device according to claim 2 or 3, wherein said second means (3,4,6) comprises beads (4) distributed along said first means (2) and contained therein.

6. A device according to claim 1 or 2, wherein said first means (2,5) comprises two longitudinal elements (5) which zig-zag towards and away from each other at said second means (3).

7. A device according to any preceding claim, and for use in acute reconstruction of a ligament, wherein said first means (2,5) terminates just beyond one end of said second means (3,4,6).

8. A device according to claim 7, and further comprising means (18) just beyond said one end of said

0249346

second means (3,4,6) providing a sewing-in location for sutures.

9. A cruciate ligament prosthesis, characterized in that the prosthesis comprises first and second prosthetic ligamentary devices (1) a section of each of which, when subjected longitudinally to an anatomical tensile force, increases in length to a limited degree and thereupon becomes virtually inextensible, and tube means (7) encircling these sections of the devices (1), fastened at the ends thereof to the devices (1) at locations beyond the sections and of material which is extensible with said sections.

10. A prosthesis according to claim 10, wherein each device (1) comprises a first means (2,5) which is relatively inelastic longitudinally of the device (1), and a second means (3,4,6) which is relatively elastic transversely of the device (1), said first means (2,5) , at said second means (3,4,6) and in one longitudinal direction of said first means (2,5), diverging outwardly and then converging inwardly, so that the subjection of the first means (2,5) to an anatomical tensile force increases the length of the first means (2,5) against the action of the second means (3,4,6) to a limited degree and thereupon said first means (2,5) becomes virtually inextensible.

1/2

0249346

FIG.1.     FIG.2.     FIG.3.

FIG.4.

FIG.7.

Fig.5.

Fig.6.